# FASCICULE DE BREVET EUROPEEN

(11) **EP 0 909 268 B1**
(45) Date de publication et mention de la délivrance du brevet: **05.06.2002**
(21) Numéro de dépôt: 98905479.6
(22) Date de dépôt: 02.02.1998
(51) Int. Cl.: C07C 46/00, C07C 46/02, C07C 50/34

(54) **PROCEDE DE PREPARATION D'ANTHRAQUINONES SUBSTITUEES, ET APPLICATION A LA PREPARATION DE RHEINES**
VERFAHREN ZUR HERSTELLUNG SUBSTITUIERTER ANTHRACHINONE UND IHRE VERWENDUNG ZUR HERSTELLUNG VON RHEINEN
METHOD FOR PREPARING SUBSTITUTED ANTHRAQUINONE AND APPLICATION TO THE PREPARATION OF RHEINS

(30) Priorité: 03.02.1997 FR 9701161
(43) Date de publication de la demande: 21.04.1999
(73) Titulaire: Girex, 29000 Quimper (FR)
(72) Inventeur: ESTANOVE, Cyril, F-92100 Boulogne (FR); PRUVOST, François, F-29000 Quimper (FR)
(74) Mandataire: L'Helgoualch, Jean
(86) Numéro de dépôt international: FR9800189
(87) Numéro de publication internationale: WO9833757

(56) Documents cités:
- WO-A-97/16404
- GB-A- 2 190 080
- KROHN K ET AL: "SYNTHESIS OF ZETA-PYRROMYCINONE, 7-DEOXYAURAMYCINONE, AND 7-DEOXYAKLAVINONE VIA KETOESTER CYCLIZATION" TETRAHEDRON, vol. 40, no. 19, pages 3677-3694, XP002008447
- KROHN K: "SYNTHETISCHE ANTHRACYCLINONE XVI 1) SYNTHESE HYDROXYLIERTER ANTHRACHINONE DURCH REGIOSELEKTIVE DIELS-ALDER-REAKTION" TETRAHEDRON LETTERS, vol. 21, no. 37, pages 3557-3560, XP002008446
- THOMPSON H W ET AL: "STEREOCHEMICAL EQUILIBRIUM IN BENZOCTALONES" JOURNAL OF ORGANIC CHEMISTRY, vol. 53, no. 18, pages 4201-4209, XP002008448
- DATABASE WPI Section Ch, Week 8744 Derwent Publications Ltd., London, GB; Class B03, AN 87-309176 XP002008450 & JP 62 126 170 A (MITSUI TOATSU CHEM INC) , 8 juin 1987
- DATABASE XFIRE BEILSTEIN INFORMATION SERVICE REF. 2198665 MITTER; DAS-GUPTA; BACHHWAT

## Description

La présente invention concerne un nouveau procédé de préparation d'anthraquinones substituées à partir de 1,4-naphtoquinones, ainsi que l'application des produits obtenus comme intermédiaires de synthèse de produits présentant des propriétés utiles en thérapeutique.

La préparation d'anthraquinones telles que le chrysophanol par addition de 6-méthoxy-4-méthyl-pyrone sur une naphtoquinone telle que la juglone, suivant la réaction de Diels-Alder, a été décrite par M. E. Jung et al., J.C.S. Chem. Comm. 95 (1978). Cependant, ce procédé nécessite plusieurs étapes, c'est-à-dire une addition suivie d'une oxydation par l'oxyde d'argent pour provoquer l'aromatisation des cycles, et une déméthylation. De plus, la réaction implique l'utilisation de diazométhane qui présente des inconvénients bien connus.

Le brevet GB-A-2.190.080 décrit un procédé de préparation d'anthraquinones par réaction d'un dérivé de butadiène sur une naphtoquinone en présence d'un catalyseur à base de métal de transition, mais ce procédé doit être mis en oeuvre dans une enceinte maintenue sous pression élevée.

Des voies de synthèses d'anthracyclinones par réaction de cyclo-addition de Diels-Alder ont aussi été décrites par M. Petrzilka et J.I. Grayson [Synthesis, 753 (1981)]. Suivant ces auteurs, la réaction d'addition regio-spécifique d'un diène sur une quinone peut être obtenue en utilisant comme catalyseur un acide de Lewis constitué par le composé BF₃-O(C₂H₅)₂.

K. Krohn et al., dans "Synthesis of zeta-pyrromycinone, 7-deoxyauramycinone, and 7-deoxyaklavinone via ketoester cyclization", Tetrahedron, vol.40, n°19, pp.3677-3694, décrivent une réaction de Diels-Alder entre une 1,4-naphtoquinone et un triméthylsiloxy-1,3-butadiène, suivie d'une oxydation par le chlorochromate de pyridinium (PCC) en présence d'acide acétique, pour former une 9,10-anthraquinone (pachybasin).

Le procédé suivant la présente invention permet de préparer des anthraquinones substituées représentées par la formule générale (I) ci-après : 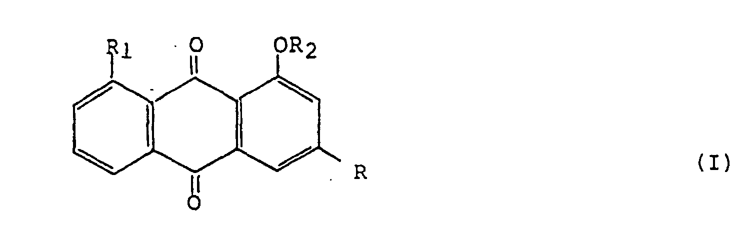 à partir de 1,4-naphtoquinones, en seulement deux étapes et avec un excellent rendement.

Dans la formule générale (I) ci-dessus, R représente un atome d'hydrogène ou un groupe alkyle linéaire ou ramifié de 1 à 5 atomes de carbone, un groupe chlorométhyle, un groupe -COCl, un groupe -COOR' ou un groupe -CH₂OR' où R' est un atome d'hydrogène ou un groupe alkyle linéaire ou ramifié de 1 à 5 atomes de carbone, R₁ représente un atome d'hydrogène, un groupe hydroxyle, alcoxy linéaire ou ramifié de 1 à 5 atomes de carbone, ou acyloxy de 1 à 5 atomes de carbone, et R₂ représente un atome d'hydrogène. Le cas échéant, on peut effectuer une acétylation pour obtenir le composé de formule (I) où R₂ est un groupe acétyle.

Conformément au procédé de l'invention, dans une première étape, on effectue une réaction de Diels-Alder entre une 1,4-naphtoquinone de formule générale (II) : 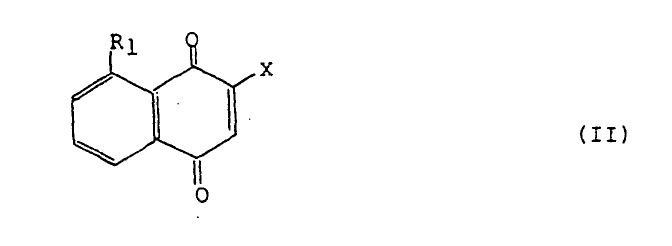 dans laquelle R₁ représente un atome d'hydrogène, un groupe hydroxyle, alcoxy linéaire ou ramifié de 1 à 5 atomes de carbone, ou acyloxy de 1 à 5 atomes de carbone, et X représente un atome d'hydrogène ou d'halogène,
et un diène acyclique de formule générale (III) :

CH₂ = CR - CH = CH - OR₃ (III)

dans laquelle R représente un atome d'hydrogène ou un groupe alkyle linéaire ou ramifié de 1 à 5 atomes de carbone, un groupe chlorométhyle, un groupe -COCl, un groupe -COOR' ou un groupe -CH₂OR' où R' est un atome d'hydrogène ou un groupe alkyle linéaire ou ramifié de 1 à 5 atomes de carbone, et R₃ représente un groupe silyle de formule -Si(R₄)₃ où R₄ est un groupe alkyle linéaire ou ramifié de 1 à 5 atomes de carbone,
pour obtenir une 1,4,4a,9a-tétrahydroanthraquinone substituée de formule générale (IV) : 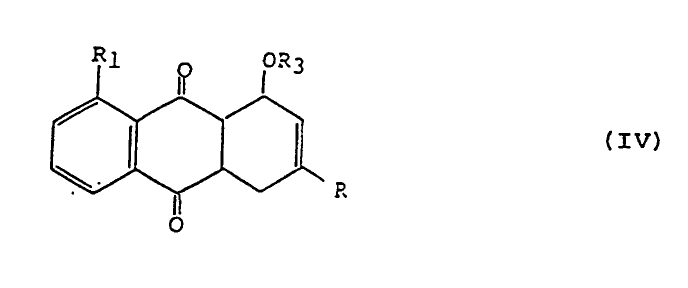 dans laquelle R, R₁ et R₃ ont les définitions indiquées ci-dessus,
puis on effectue, sur la tétrahydroanthraquinone de formule générale (IV), une réaction de déprotection oxydante au moyen du réactif de Jones, pour obtenir l'anthraquinone recherchée, représentée par la formule générale (I) indiquée ci-dessus.

La réaction de déprotection oxydante s'effectue de préférence par action d'un mélange de dichromate de potassium et d'acide sulfurique aqueux dans un solvant tel l'acide acétique, l'éther éthylique, du diméthylsulfoxyde ou du dichlorométhane en présence d'un agent de transfert de phase tel qu'un sel d'ammonium quaternaire, ou encore une cétone, et de préférence l'acétone.

Dans la formule (II) ci-dessus représentant la naphtoquinone de départ, de préférence, R₁ représente un groupe hydroxyle ou acétoxy, et X est un atome d'hydrogène ou un atome de chlore. Dans la formule générale (III) représentant le diène acyclique, il est préférable que R₃ représente un groupe triméthylsilyle, et R un atome d'hydrogène ou un groupe méthyle.

Le diène acyclique utilisé dans la réaction décrite ci-dessus peut être un dérivé du butadiène, tel que par exemple le 1-(triméthylsilyloxy)-1,3-butadiène et le 3-méthyl-1-(triméthylsilyloxy)-1,3-butadiène.

Parmi les naphtoquinones de formule générale (II), on utilise de préférence la juglone, représentée par la formule (II) où R₁ représente un groupe hydroxyle, ou la 3-chlorojuglone, représentée par la même formule où X est un atome de chlore. La juglone peut être préparée par exemple par oxydation du 1,5-dioxynaphtalène en présence d'un catalyseur approprié comme décrit dans le brevet SU-1.817.767, ou par l'oxyde de chrome par la méthode de G. Jesaitis et al., J, Chem, Ed., 49, 436 (1972), ou encore par oxydation au moyen d'oxygène en présence d'un catalyseur à base de cobalt tel que la salcomine, suivant la méthode de T. Wakamatsu et al., Synthetic Communications, 14, 1167 (1984).

La réaction de cyclo-addition de Diels-Alder entre la 1,4-naphtoquinone de formule générale (II) et le diène acyclique de formule générale (III) s'effectue de préférence dans un solvant qui peut être choisi parmi les solvants hydrocarbonés et les alcools, tel que le toluène, le benzène ou le méthanol. Suivant une forme avantageuse de réalisation de l'invention, la réaction est effectuée en présence d'une quantité catalytique d'hydroquinone. Il peut également être avantageux d'effectuer la réaction en présence d'un catalyseur de Lewis choisi parmi, par exemple, le chlorure de zinc, le chlorure ferrique et le triacétate de bore.

La réaction d'addition s'effectue de préférence à température ambiante ou en chauffant légèrement à une température comprise entre 20 et 50°C.

Comme indiqué ci-dessus, on effectue une réaction de déprotection oxydante sur la tétrahydroanthraquinone de formule générale (IV), au moyen du réactif de Jones, ce qui permet tout à la fois de provoquer la désilylation, l'oxydation et l'aromatisation en une seule étape, pour obtenir l'anthraquinone recherchée représentée par la formule générale (I) avec un bon rendement. Cette réaction peut s'effectuer avantageusement à froid, et de préférence à la température de 0°C environ, dans un solvant approprié, en une seule étape, sans qu'il soit nécessaire d'isoler de produit intermédiaire contrairement aux procédés connus. Par exemple, suivant les techniques connues (voir K. Krohn, Liebigs Ann. Chem. (1981) p. 2285-2297), on peut préparer du chrysophanol en trois étapes à partir d'une napthoquinone sur laquelle on effectue une réaction de Diels-Alder pour obtenir une tétrahydroanthraquinone qui doit ensuite être traitée par hydrolyse pour provoquer la désilylation, puis on effectue un traitement par un oxydant à base de chrome tel que le chlorochromate de pyridinium pour pouvoir obtenir le produit recherché sous forme de mélange de chrysophanol et d'isochrysophanol.

Le procédé conforme à la présente invention est particulièrement avantageux en ce qu'il permet d'obtenir aisément l'anthraquinone recherchée, c'est-à-dire un composé à cycles aromatiques, sans utilisation de composés tel que l'oxyde d'argent pour provoquer l'aromatisation, contrairement aux schémas réactionnels classiques. De plus, le procédé de la présente invention permet d'obtenir des anthraquinones substituées, par exemple le chrysophanol, exempts d'isomères, avec un bon rendement tout en diminuant les quantités de dérivés de chrome utilisés.

Les anthraquinones substituées obtenues par le procédé suivant la présente invention peuvent être utilisées dans la préparation de rhéines de formule générale (V) 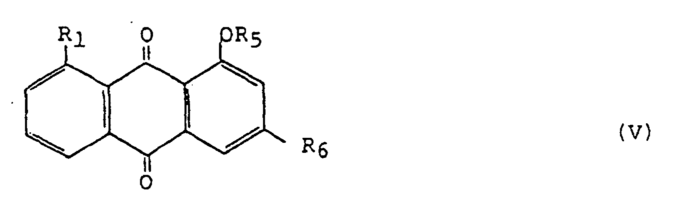 dans laquelle R₅ représente un groupe acétyle et R₆ représente un groupe -CO₂R' où R' est un atome d'hydrogène ou un groupe alkyle linéaire ou ramifié de 1 à 5 atomes de carbone, que l'on obtient en effectuant une acétylation des anthraquinones substituées de formule générale (I), suivie si nécessaire d'une oxydation et d'une purification.

Ces rhéines sont utiles en thérapeutique humaine et vétérinaire comme principes actifs de médicaments, notamment comme anti-inflammatoires non stéroïdiens pour le traitement de l'arthrite et de l'arthrose.

Les exemples suivants illustrent l'invention plus en détail sans en limiter la portée.

### Exemple 1

Dans un ballon de 25 ml, on fait réagir à température ambiante et sous atmosphère d'azote, un mélange de 0,4 g de 5-hydroxy-1,4-naphtoquinone (2,3.10⁻³ mole) et de 0,47 g de 3-méthyl-1-(triméthylsilyloxy)-1,3-butadiène (3,0.10⁻³ mole) dans 5 ml de dichlorométhane ou de toluène, en présence d'hydroquinone en quantité catalytique. L'agitation est maintenue pendant environ 14 heures.

L'évolution de la réaction est suivie par chromatographie sur couche mince. La réaction est terminée lorsque la juglone n'est plus détectée.

Le solvant est éliminé par distillation sous pression réduite. Après précipitation par un minimum de pentane, le solide est filtré et on obtient 728 mg d'un mélange constitué de 90% de 8-hydroxy-3-méthyl-1-(triméthylsilyloxy)-1,4,4a,9a tétrahydro-9,10-anthraquinone (1,98.10⁻³ mole) et de 10% de 5-hydroxy-3-méthyl-1-(triméthylsilyloxy)-1,4,4a,9a-tétrahydro-9,10-anthraquinone (0,22.10⁻³ mole).

Les adduits de Diels-Alder sont ensuite agités en présence de 8.5 ml de réactif de Jones (1.67 g de dichromate de potassium (5,68.10⁻³ mole) + 7 ml d'eau + 1.3 ml d'acide sulfurique concentré) dans 70 ml d'acétone. La réaction est totale après 15 minutes à 0°C. L'oxydant en excès est détruit par de l'isopropanol avant de filtrer les sels de chrome sur célite. L'acétone est évaporée afin de permettre au chrysophanol et à l'isochrysophanol de précipiter. Après filtration, on obtient 527 mg d'un mélange constitué de 90% de chrysophanol (1,87.10⁻³ mole) et de 10% d'isochrysophanol (0,2.10⁻³ mole), ce qui équivaut à un rendement global de 90%.

### Exemple 2

On procède comme dans l'exemple 1, mais après la réaction de Diels-Alder, le solvant est éliminé par distillation sous pression réduite, et une cristallisation dans l'éther de pétrole permet d'obtenir 531 mg de 8-hydroxy-3-méthyl-1-(triméthylsilyloxy)-1,4,4a,9a-tétrahydro-9,10-anthraquinone (1,6.10⁻³ mole) sans présence de l'autre isomère.

L'adduit de Diels-Alder est ensuite traité par 6.2 ml de réactif de Jones afin d'obtenir après traitement, 384 mg de chrysophanol (1,51.10⁻³ mole), ce qui équivaut à un rendement global de 66% en chrysophanol.

### Exemple 3

On procède comme dans l'exemple 1 mais en ajoutant 0.1 équivalent de B(OAc)₃ comme catalyseur en tant qu'acide de Lewis lors de la réaction de Diels-Alder.

La réaction se déroule de la même manière et la juglone n'est plus détectée par chromatographie sur couche mince après une nuit d'agitation.

Le solvant est éliminé par distillation sous pression réduite. Après précipitation par un minimum de pentane, le solide est filtré et on obtient 728 mg d'un mélange constitué de 95% de 8-hydroxy-3-méthyl-1-(triméthylsilyloxy)-1,4,4a,9a tétrahydro-9,10-anthraquinone (2,09.10⁻³ mole) et de 5% de 5-hydroxy-3-méthyl-1-(triméthylsilyloxy)-1,4,4a,9a-tétrahydro-9,10-anthraquinone (0,11.10⁻³ mole).

Les adduits de Diels-Alder sont ensuite traités par 8.5 ml de réactif de Jones afin d'obtenir après traitement 527 mg d'un mélange constitué de 95% de chrysophanol (1,97.10⁻³ mole) et de 5% d'isochrysophanol (0,10.10⁻³ mole), ce qui équivaut à un rendement global de 90%.

### Exemple 4

On procède comme dans l'exemple 1, mais en utilisant seulement 2,8 ml de réactif dé Jones.

Après traitement, on obtient 460 mg d'un mélange constitué de 90% de chrysophanol (1,63.10⁻³ mole) et de 10% d'isochrysophanol (0,18.10⁻³ mole), ce qui équivaut à un rendement global de 79%.

### Exemple 5

On procède comme dans l'exemple 2, mais en utilisant 2,8 ml de réactif de Jones.

Après traitement, on obtient 335 mg de chrysophanol (1,32.10⁻³ mole), ce qui équivaut à un rendement global de 57% en chrysophanol.

### Exemple 6

On procède comme dans l'exemple 1, mais en faisant réagir 0,36 g de 1,4-naphtoquinone (2,3.10⁻³ mole) et 0.47 g de 3-méthyl-1-(triméthylsilyloxy)-1,3-butadiène (3,0.10⁻³ mole).

Après réaction avec le réactif de Jones et traitement, on obtient 494 mg de 1-hydroxy-3-méthyl-9,10-anthraquinone (2,07.10⁻³ mole), ce qui équivaut à un rendement global de 90%.

### Exemple 7

On procède comme dans l'exemple 1, mais en faisant réagir 0.36 g de 1,4-naphtoquinone (2,3.10⁻³ mole) et 0,47 g de 3-méthyl-1-(triméthylsilyloxy)-1,3-butadiène (3,0.10⁻³ mole), et en utilisant seulement 2,8 ml de réactif de Jones.

Après réaction avec le réactif de Jones et traitement, on obtient 431 mg de 1-hydroxy-3-méthyl-9,10-anthraquinone (1,81.10⁻³ mole), ce qui équivaut à un rendement global de 79%.

### Exemple 8

On procède comme dans l'exemple 1, mais en faisant réagir 0,4 g de 5-hydroxy-1,4-naphtoquinone (2,3.10⁻³ mole) et 0,42 g de 1-(triméthylsilyloxy)-1,3-butadiène (3,0.10⁻³ mole).

Après réaction avec le réactif de Jones et traitement, ont obtient 498 mg d'un mélange constitué de 90% de 1,8-dihydroxy-9,10-anthraquinone (1,87.10⁻³ mole) et de 10% de 1,5-dihydroxy-9,10-anthraquinone (0,2.10⁻³ mole), ce qui équivaut à un rendement global de 90%.

### Exemple 9

On procède comme dans l'Exemple 1, mais en ajoutant 63 mg de chlorure de zinc comme catalyseur en tant qu'acide de Lewis dans la première étape.

La réaction se déroule de la même manière et la juglone n'est plus détectée par chromatographie sur couche mince au bout de 14 heures.

On obtient ainsi 520 mg d'un mélange constitué de 60% de 8-hydroxy-3-méthyl-1-(triméthylsilyloxy)-1,4,4a,9a-tétrahydroanthraquinone et de 40% de 8-hydroxy-2-méthyl-4-triméthylsilyloxy-1,4,4a,9a-tétrahydroanthraquinone.

Ces deux isomères sont traités sous agitation par le même réactif de Jones que dans l'Exemple 1, dans l'acétone. Après élimination de l'oxydant en excès, filtration des sels de chrome et évaporation du solvant, on obtient un mélange de 60% de chrysophanol et 40% d'isochrysophanol avec un rendement global de 64%.

### Exemple 10 (Exemple comparatif)

Dans un ballon de 5 ml, on fait réagir à température ambiante un mélange de 0,87 g de 5-hydroxy-1,4-naphtoquinone, et de 0,94 g de 3-méthyl-1-(triméthylsilyloxy)-1,3-butadiène dans 3 ml de dichlorométhane, pendant environ 12 heures.

L'évolution de la réaction est suivie par chromatographie sur couche mince. La réaction est terminée lorsque la juglone n'est plus détectée.

On obtient ainsi un mélange constitué de 90% de 8-hydroxy-3-méthyl-1-(triméthylsilyloxy)-1,4,4a,9a-tétrahydroanthraquinone et de 10% de 8-hydroxy-2-méthyl-4-(triméthylsilyloxy)-1,4,4a,9a-tétrahydroanthraquinone.

Les deux isomères en mélange sont désilylés en milieu acide (HCl 1N, 0,5 ml dans 5 ml de méthanol), puis oxydés et aromatisés à l'aide de chlorochromate de pyridinium (2,36 g) dans 100 ml de dichlorométhane, en maintenant le mélange sous agitation pendant environ 4 heures. Après addition de 2 g de sulfate de magnésium et filtration, puis évaporation du solvant sous pression réduite, on obtient un mélange de chrysophanol et d'isochrysophanol.

Le mélange chrysophanol-isochrysophanol ainsi obtenu avec un rendement de 50% est identifié par RMN.

Cet exemple montre que le rendement est nettement inférieur quand on procède en trois étapes, suivant la technique classique, sans utilisation du réactif de Jones.

## Revendications

1. Procédé de préparation d'anthraquinones substituées représentées par la formule générale (I) ci-dessous 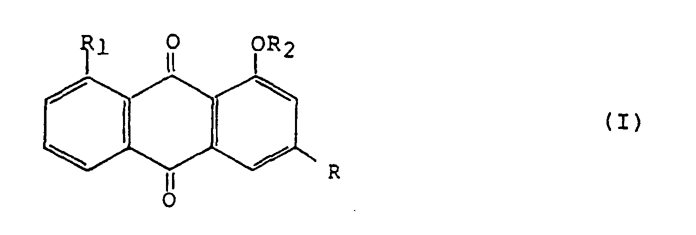 dans laquelle R représente un atome d'hydrogène ou un groupe alkyle linéaire ou ramifié de 1 à 5 atomes de carbone, un groupe chlorométhyle, un groupe -COCl, un groupe -COOR' ou un groupe -CH₂OR' où R' est atome d'hydrogène ou un groupe alkyle linéaire ou ramifié de 1 à 5 atomes de carbone, R₁ représente un atome d'hydrogène, un groupe hydroxyle, alcoxy linéaire ou ramifié de 1 à 5 atomes de carbone, ou acyloxy de 1 à 5 atomes de carbone, et R₂ représente un atome d'hydrogène
**caractérisé en ce que** l'on effectue une réaction de Diels-Alder entre une 1,4-naphtoquinone de formule générale (II) : 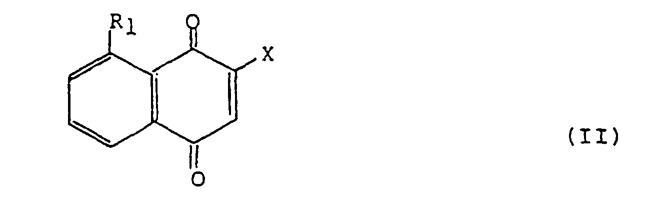 dans laquelle R₁ représente un atome d'hydrogène, un groupe hydroxyle, alcoxy linéaire ou ramifié de 1 à 5 atomes de carbone, ou acyloxy de 1 à 5 atomes de carbone, et X représente un atome d'hydrogène ou d'halogène,
et un diène acyclique de formule (III) :
CH₂ = CR - CH = CH - OR₃ (III)
dans laquelle R représente un atome d'hydrogène ou un groupe alkyle linéaire ou ramifié de 1 à 5 atomes de carbone, un groupe chlorométhyle, un groupe -COCl, un groupe -COOR' ou un groupe -CH₂OR' où R' est un atome d'hydrogène ou un groupe alkyle linéaire ou ramifié de 1 à 5 atomes de carbone, et R₃ représente un groupe silyle de formule -Si(R₄)₃ où R₄ est un groupe alkyle linéaire ou ramifié de 1 à 5 atomes de carbone,
pour obtenir une tétrahydroanthraquinone substituée de formule générale (IV) : 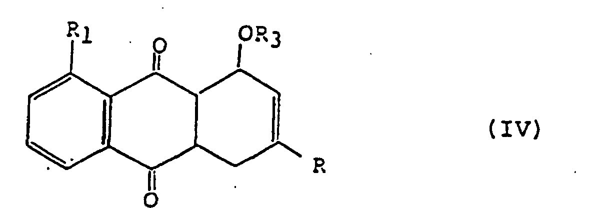 dans laquelle R, R₁ et R₃ ont les définitions indiquées ci-dessus,
puis on effectue, sur la tétrahydroanthraquinone de formule générale (IV), une réaction de déprotection oxydante au moyen du réactif de Jones, pour obtenir l'anthraquinone de formule générale (I)

2. Procédé selon la revendication 1, **caractérisé en ce que** la réaction est effectuée en présence d'un acide de Lewis.

3. Procédé selon la revendication 2, **caractérisé en ce que** l'acide de Lewis est choisi parmi le chlorure de zinc, le chlorure ferrique, et le triacétate de bore.

4. Procédé selon la revendication 1, **caractérisé en ce que** la réaction est effectuée en présence d'hydroquinone en quantité catalytique.

5. Procédé selon l'une quelconque des revendications précédentes, **caractérisé en ce que** R₁ représente un groupe hydroxyle ou acétoxy, et X est un atome d'hydrogène ou un atome de chlore.

6. Procédé selon l'une quelconque des revendications précédentes, **caractérisé en ce que** R représente un atome d'hydrogêne ou un groupe méthyle, et R₃ représente un groupe triméthylsilyle.

7. Procédé selon la revendication 6, **caractérisé en ce que** le diène de formule (III) est le 1-(trimethylsilyloxy)-1,3-butadiène ou le 3-méthyl-1-(triméthylsilyloxy)-1,3-butadiène.

8. Procédé selon la revendication 5, **caractérisé en ce que** la naphtoquinone de formule générale (II) est la juglone ou la 3-chlorojuglone.

9. Procédé selon l'une quelconque des revendications précédentes, **caractérisé en ce que** la réaction de déprotection oxydante de la tétrahydroanthraquinone de formule (IV) est réalisée au moyen d'un mélange à base de dichromate de potassium et d'acide sulfurique.

10. Procédé selon la revendication 9, **caractérisé en ce que** la réaction de déprotection oxydante s'effectue dans un solvant choisi parmi l'acide acétique, l'éther éthylique, le diméthylsulfoxyde, le dichlorométhane ou une cétone.

## Claims

1. Process for the preparation of substituted anthraquinones represented by the general formula (I) hereinbelow 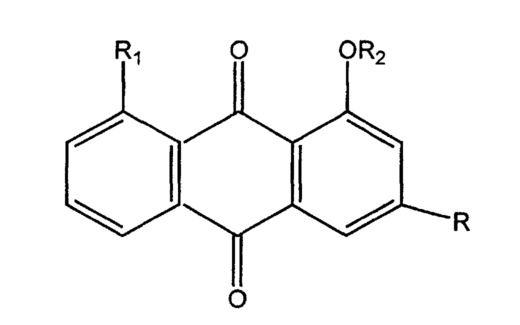 in which R represents a hydrogen atom or a linear or branched alkyl group comprising 1 to 5 carbon atoms, a chloromethyl group, a -COCl group, a -COOR' group or a -CH₂OR' group where R' is a hydrogen atom or a linear or branched alkyl group comprising 1 to 5 carbon atoms, R₁ represents a hydrogen atom, a hydroxyl group, a linear or branched alkoxy group comprising 1 to 5 carbon atoms or an acyloxy group comprising 1 to 5 carbon atoms and R₂ represents a hydrogen atom,
**characterized in that** a Diels-Alder reaction is carried out between a 1,4-naphthoquinone of general formula (II): 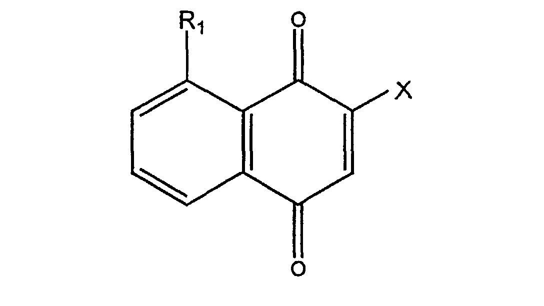 in which R₁ represents a hydrogen atom, a hydroxyl group, a linear or branched alkoxy group comprising 1 to 5 carbon atoms or an acyloxy group comprising 1 to 5 carbon atoms and X represents a hydrogen or halogen atom,
and an acyclic diene of formula (III):
CH₂=CR-CH=CH-OR₃ (III)
in which R represents a hydrogen atom or a linear or branched alkyl group comprising 1 to 5 carbon atoms, a chloromethyl group, a -COCl group, a -COOR' group or a -CH₂OR' group where R' is a hydrogen atom or a linear or branched alkyl group comprising 1 to 5 carbon atoms and R₃ represents a silyl group of formula -Si(R₄)₃ where R₄ is a linear or branched alkyl group comprising 1 to 5 carbon atoms,
in order to obtain a substituted tetrahydroanthraquinone of general formula (IV): 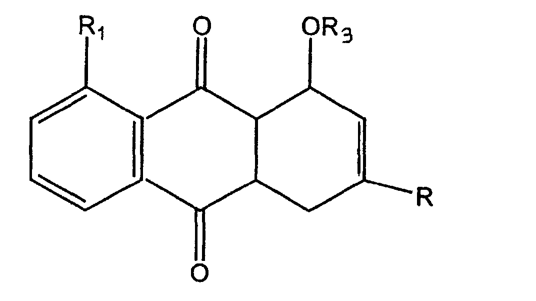 in which R, R, and R₃ have the definitions indicated hereinabove,
and then an oxidizing deprotection reaction is carried out, by means of the Jones reagent, on the tetrahydroanthraquinone of general formula (IV), in order to obtain the anthraquinone of general formula (I).

2. Process according to Claim 1, **characterized in that** the reaction is carried out in the presence of a Lewis acid.

3. Process according to Claim 2, **characterized in that** the Lewis acid is selected from zinc chloride, ferric chloride and boron triacetate.

4. Process according to Claim 1, **characterized in that** the reaction is carried out in the presence of a catalytic amount of hydroquinone.

5. Process according to any one of the preceding claims, **characterized in that** R₁ represents a hydroxyl or acetoxy group and X is a hydrogen atom or a chlorine atom.

6. Process according to any one of the preceding claims, **characterized in that** R represents a hydrogen atom or a methyl group and R₃ represents a trimethylsilyl group.

7. Process according to Claim 6, **characterized in that** the diene of formula (III) is 1-(trimethylsilyloxy)-1,3-butadiene or 3-methyl-1-(trimethylsilyloxy)-1,3-butadiene.

8. Process according to Claim 5, **characterized in that** the naphthoquinone of general formula (II) is juglone or 3-chlorojuglone.

9. Process according to any one of the preceding claims, **characterized in that** the oxidizing deprotection reaction of the tetrahydroanthraquinone of formula (IV) is carried out by means of a mixture based on potassium dichromate and on sulfuric acid.

10. Process according to Claim 9, **characterized in that** the oxidizing deprotection reaction is carried out in a solvent selected from acetic acid, ethyl ether, dimethyl sulfoxide, dichloromethane or a ketone.

## Patentansprüche

1. Verfahren zur Herstellung von substituierten Anthrachinonen der nachstehenden allgemeinen Formel (I): 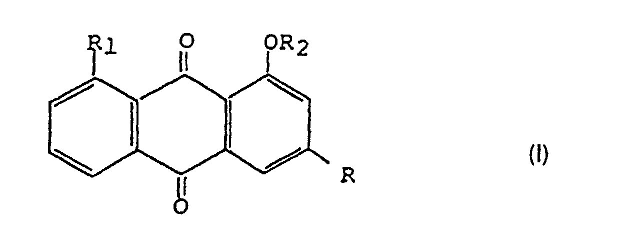 worin R für ein Wasserstoffatom oder eine unverzweigte oder verzweigte Alkylgruppe mit 1 bis 5 Kohlenstoffatomen, eine Chlormethylgruppe, eine Gruppe -COCI, eine Gruppe -COOR' oder eine Gruppe -CH₂OR' steht, worin R' ein Wasserstoffatom oder eine unverzweigte oder verzweigte Alkylgruppe mit 1 bis 5 Kohlenstoffatomen ist, R₁ für ein Wasserstoffatom, eine Hydroxygruppe, unverzweigtes oder verzweigtes Alkoxy mit 1 bis 5 Kohlenstoffatomen oder Acyloxy mit 1 bis 5 Kohlenstoffatomen steht, und R₂ für ein Wasserstoffatom steht,
**dadurch gekennzeichnet, dass** eine Diels-Alder-Reaktion zwischen einem 1,4-Naphtochinon der allgemeinen Formel (II): 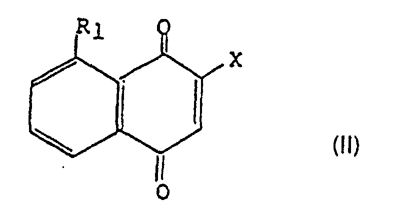 worin R₁ für ein Wasserstoffatom, eine Hydroxygruppe, unverzweigtes oder verzweigtes Alkoxy mit 1 bis 5 Kohlenstoffatomen oder Acyloxy mit 1 bis 5 Kohlenstoffatomen steht, und X für ein Wasserstoffatom oder ein Halogenatom steht,
und einem azyklischen Dien der Formel (III):
CH₂ = CR - CH = CH - OR₃ (III)
bewirkt wird, worin R für ein Wasserstoffatom oder eine unverzweigte oder verzweigte Alkylgruppe mit 1 bis 5 Kohlenstoffatomen, eine Chlormethylgruppe, eine Gruppe -COCl, eine Gruppe -COOR' oder eine Gruppe -CH₂OR' steht, worin R' ein Wasserstoffatom oder eine unverzweigte oder verzweigte Alkylgruppe mit 1 bis 5 Kohlenstoffatomen ist, und R₃ für eine Silylgruppe der Formel -Si(R₄)₃ steht, worin R₄ ein unverzweigte oder verzweigte Alkylgruppe mit 1 bis 5 Kohlenstoffatomen ist,
um ein substituiertes Tetrahydroanthrachinon der allgemeinen Formel (IV): 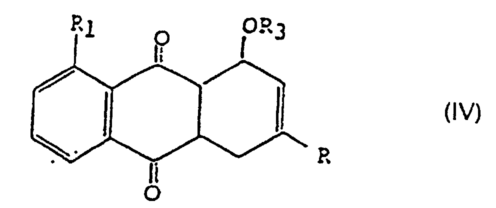 zu erhalten, worin R, R₁ und R₃ die oben angeführte Bedeutung haben,
und dann am Tetrahydroanthrachinon der allgemeinen Formel (IV) eine Oxidationsreaktion zum Entfernen der Schutzgruppe mit Hilfe eines Jones-Reagens bewirkt wird, um das Anthrachinon der allgemeinen Formel (I) zu erhalten.

2. Verfahren nach Anspruch 1, **dadurch gekennzeichnet, dass** die Reaktion in Gegenwart einer Lewis-Säure durchgeführt wird.

3. Verfahren nach Anspruch 2, **dadurch gekennzeichnet, dass** die Lewis-Säure aus Zinkchlorid, Eisen(III)-chlorid und Bortriacetat ausgewählt ist.

4. Verfahren nach Anspruch 1, **dadurch gekennzeichnet, dass** die Reaktion in Gegenwart von Hydrochinon in katalytischer Menge durchgeführt wird.

5. Verfahren nach einem der vorangegangenen Ansprüche, **dadurch gekennzeichnet, dass** R₁ für eine Hydroxy- oder Acetoxygruppe steht und X ein Wasserstoffatom oder ein Chloratom ist.

6. Verfahren nach einem der vorangegangenen Ansprüche, **dadurch gekennzeichnet, dass** R für ein Wasserstoffatom oder eine Methylgruppe steht und R₃ für eine Trimethylsilylgruppe steht.

7. Verfahren nach Anspruch 6, **dadurch gekennzeichnet, dass** das Dien der Formel (III) 1-(Trimethylsilyloxy)-1,3-butadien oder 3-Methyl-1-(trimethylsilyloxy)-1,3-butadien ist.

8. Verfahren nach Anspruch 5, **dadurch gekennzeichnet, dass** das Naphthochinon der allgemeinen Formel (II) Juglon oder 3-Chlorjuglon ist.

9. Verfahren nach einem der vorangegangenen Ansprüche, **dadurch gekennzeichnet, dass** die Oxidationsreaktion zum Entfernen der Schutzgruppe vom Tetrahydroanthrachinon der Formel (IV) mit Hilfe eines Gemisches auf Basis von Kaliumdichromat und Schwefelsäure durchgeführt wird.

10. Verfahren nach Anspruch 9, **dadurch gekennzeichnet, dass** die Oxidationsreaktion zum Entfernen der Schutzgruppe in einem Lösungsmittel durchgeführt wird, das aus Essigsäure, Ethylether, Dimethylsulfoxid, Dichlormethan oder einem Keton ausgewählt ist.
